(19) Europäisches Patentamt
European Patent Office
Office européen des brevets

(11) EP 1 842 555 A1

(12) EUROPEAN PATENT APPLICATION
published in accordance with Art. 158(3) EPC

(43) Date of publication:
10.10.2007 Bulletin 2007/41

(51) Int Cl.:
A61K 45/00 (2006.01)          A61K 31/485 (2006.01)
A61K 31/404 (2006.01)          A61K 38/00 (2006.01)
A61P 25/04 (2006.01)

(21) Application number: 05820197.1

(22) Date of filing: 26.12.2005

(86) International application number:
PCT/JP2005/023783

(87) International publication number:
WO 2006/070742 (06.07.2006 Gazette 2006/27)

(84) Designated Contracting States:
AT BE BG CH CY CZ DE DK EE ES FI FR GB GR
HU IE IS IT LI LT LU LV MC NL PL PT RO SE SI
SK TR

(30) Priority: 27.12.2004 JP 2004376533

(71) Applicants:
• Kyoto University
  Kyoto-shi, Kyoto 606-8501 (JP)
• Fukuoka University
  Junan-ku, Fukuoka-shi
  Fukuoka 814-0180 (JP)
• Japan Health Sciences Foundation
  Tokyo 103-0001 (JP)

(72) Inventors:
• TSUJIMOTO, G.,
  c/o Graduate Sch. Pharmaceut. Sci.
  Sakyo-ku,
  Kyoto-shi,
  Kyoto 606-8501 (JP)

• TAKANO, Y.,
  c/o Fukuoka University
  Fukuoka-shi,
  Fukuoka 814-0180 (JP)
• HONDA, Kenji,
  c/o Fukuoka University
  Fukuoka-shi,
  Fukuoka 814-0180 (JP)
• TANOUE, Akito,
  c/o N.C.C.H.D.
  Tokyo 157-8535 (JP)

(74) Representative: Polypatent
An den Gärten 7
51491 Overath (DE)

(54) **TOLERANCE DEVELOPMENT INHIBITOR FOR NARCOTIC ANALGESIC AGENT**

(57) A medicament for suppressing development of tolerance to analgesic effect induced by administration of a narcotic analgesic such as morphine, which comprises an antagonist of the vasopressin receptor 1b as an active ingredient.

Fig. 6

# P < 0.02 vs. vehicle
** P < 0.01 vs. day1

EP 1 842 555 A1

**Description**

Technical Field

The present invention relates to a medicament for suppressing development of tolerance to a narcotic analgesic such as morphine.

Background Art

Since narcotic analgesics such as morphine have superior analgesic effect on visceral pain and the like, they have been clinically used for pain treatment of patients with terminal cancer. However, prolonged administration of narcotic analgesics rapidly induces tolerance to the analgesic effect as their primary action. Therefore, careful control of administration frequency and dose thereof is required to minimize the development of tolerance while achieving desired analgesic effect. As agents for suppressing development of tolerance to narcotic analgesics, for example, medicaments described in International Patent Publication WO97/6139 and the like have been proposed. However, any medicament having superior effectiveness has not yet been clinically developed.

Vasopressin is an antidiuretic hormone which is a peptide consisting of nine amino acids. In many mammals including human, the substance exists as arginine vasopressin (AVP) in which the eighth amino acid is arginine. Vasopressin receptors are seven-transmembrane receptors belonging to the G-protein coupled receptor superfamily. As the vasopressin receptors, the $V_2$ receptor, which promotes the production of cAMP, and the $V_1$ receptor, which activates phospholipase C, increases the intracellular calcium concentration via release of inositol 1,4,5-trisphosphate and produces diacylglycerol to activate protein kinase C, are known. The $V_1$ receptor exists in the vascular smooth muscles and causes vasoconstriction via elevation of the intracellular calcium concentration.

As the $V_1$ receptors, $V_{1a}$ and $V_{1b}$ receptors are also known to exist. The $V_{1a}$ receptor is known to be involved in the vasoconstrictive action, and $V_{1b}$ receptor is known to be involved in secretion of adrenocorticotrpic hormone (ACTH) from the pituitary gland. However, many functions of the $V_{1b}$ receptor remain unrevealed. Further, no report has been made to date that teaches involvement of the $V_{1b}$ receptor in the development of tolerance to narcotic analgesics. As compounds having an suppressing action on the $V_{1b}$ receptor, those described in International Patent Application Unexamined Publication in Japanese (Kohyo) Nos. 2003-523351, 2003-523354, 2003-525287 and 2004-50265 are known. However, these publications do not suggest or teach that these compounds suppress the development of tolerance to narcotic analgesics.

Disclosure of the Invention

Object to be Achieved by the Invention

An object of the present invention is to provide a medicament having a suppressing action against development of tolerance to analgesic effect which is induced by administration of a narcotic analgesic such as morphine.

Means for Achieving the Object

The inventors of the present invention conducted various researches to achieve the foregoing object. As a result, they found that, among the vasopressin receptors, the $V_{1b}$ receptor was involved in the development of tolerance to narcotic analgesics, and antagonists of the $V_{1b}$ receptor markedly suppressed the development of tolerance to narcotic analgesics. The present invention was accomplished on the basis of the aforementioned findings.

The present invention thus provides a medicament for suppressing development of tolerance to analgesic effect of a narcotic analgesic, which comprises an antagonist of vasopressin receptor 1b as an active ingredient. According to a preferred embodiment, the present invention provides the aforementioned medicament, which is for combination use with a narcotic analgesic. The combination use can be attained by using a single dosage unit containing both of the drugs or separate dosage units each containing either of the drugs as an active ingredient. When the combination use is attained by using separate dosage units, they can be administered simultaneously or at different times. Further, the present invention provides the aforementioned medicament, wherein the narcotic analgesic is morphine hydrochloride or morphine nitrate, preferably morphine hydrochloride.

In addition to the above invention, there are provided a method for suppressing development of tolerance to analgesic effect of a narcotic analgesic, which comprises the step of administering an effective amount of vasopressin receptor 1b to a mammal including human, and use of the vasopressin receptor 1b for the manufacture of the aforementioned medicament.

Effect of the Invention

The medicament of the present invention has a suppressing action against development of tolerance to analgesic effect induced by administration of a narcotic analgesic such as morphine, and can reduce or prevent the development of tolerance to analgesic effect of a narcotic analgesic.

Brief Description of the Drawings

[Fig. 1] A graph showing changes over time in development of tolerance to morphine in $V_{1a}$ receptor knockout mice, $V_{1b}$ receptor knockout mice and control mice.
[Fig. 2] Graphs showing the suppressing action of a non-selective $V_1$ receptor antagonist (dPenTyr(Me)AVP) on development of tolerance to morphine.
[Fig. 3] Graphs showing the suppressing action of an antagonist highly selective to the $V_{1a}$ receptor (PhAcALVP) on development of tolerance to morphine.
[Fig. 4] Graphs showing the suppressing action of an antagonist selective to the $V_{1a}$ receptor (d(CH$_2$)$_5$Tyr(Me)AVP) on development of tolerance to morphine.
[Fig. 5] A graph showing changes over time in morphine-induced analgesic effect in ddy mice intracerebroventricularly administered with a $V_{1b}$ receptor antagonist.
[Fig. 6] A graph showing effect (AUC) of an intracerebroventricularly administered $V_{1b}$ receptor antagonist on development of tolerance to morphine-induced analgesic effect.

Best Mode for Carrying out the Invention

The medicament of the present invention is for suppressing development of tolerance to analgesic effect of a narcotic analgesic, which comprises an antagonist of the vasopressin receptor 1b (hereinafter, referred to as "$V_{1b}$ receptor") as an active ingredient. As the $V_{1b}$ receptor, a receptor having affinity for arginine vasopressin is preferred.
As the antagonist of the $V_{1b}$ receptor, although an antagonist selective to the $V_{1b}$ receptor is preferably used, a substance that also acts as an antagonist of the $V_{1a}$ receptor as well as to the $V_{1b}$ receptor can be used as the active ingredient of the medicament of the present invention. The affinity for the $V_{1b}$ receptor can be confirmed by, for example, the method of Y. De Keyser et al. (Febs Letters, 356, pp.215-220, 1994). Further, the antagonistic action on the $V_{1b}$ receptor can be confirmed according to, for example, the method of C.S-L., GAL (J. Pharm. Exp. Ther., 300, pp.1122-1130, 2002). Any arbitrary substance for which the antagonistic action on the $V_{1b}$ receptor is confirmed by the aforementioned method can be used as the active ingredient of the medicament of the present invention.
More specific examples of the antagonist of the $V_{1b}$ receptor include the compounds described in International Patent Application Unexamined Publication in Japanese (Kohyo) Nos. 2003-523351, 2003-523354, 2003-525287, 2004-502654 and the like. However, substances that can be used as the active ingredient of the medicament of the present invention are not limited to those described in the aforementioned publications. As the active ingredient of the medicament of the present invention, compounds in free forms or physiologically acceptable salts, or hydrates or solvates thereof may be used. Stereoisomers such as optically active substances and diastereomers, arbitrary mixtures of stereoisomers, racemates and the like may also be used as the active ingredient of the medicament of the present invention.
The medicament of the present invention can reduce or prevent development of tolerance to analgesic effect of a narcotic analgesic. The medicament of the present invention can be prophylactically used for the purpose of reducing or preventing the development of tolerance. The medicament of the present invention also has an action of reducing or eliminating tolerance to analgesic effect already developed due to administration of a narcotic analgesic. Therefore, the medicament of the present invention can also be therapeutically used for the purpose of reducing or eliminating already developed tolerance, generally with continuously using a narcotic analgesic in combination. The terminology "suppressing development of tolerance" used in the specification is meant to include reduction or elimination of already developed tolerance as described above, and should not be construed in any limitative sense.
Types of narcotic analgesics are not particularly limited so long as tolerance to their analgesic effect is substantially developed by a single administration or continuous administration over a short or prolonged period. Examples of the narcotic analgesics include morphines obtained from opium and semisynthesized products thereof, non-natural compounds such as pethidine having a morphine-like action and salts thereof, and the like.
More specific examples include alkaloids obtained from opium and semisynthesized products thereof, such as phenanthrenes (morphine, oxymorphone, hydromorphone, codeine, hydrocodeine, heroin, thebaine, buprenorphine and the like); phenylpiperidines (meperidine, fentanyl and the like); phenylheptylamines (methadone, propoxyphene and the like); morphinans (levorphanol, methorphan, levorphan and the like); benzomorphans (phenazocine, pentazocine and the like), and the like.

Further, examples also include analgesic peptides including endogenous morphine-like substances such as enkephalins (methionine enkephalin, leucine enkephalin); endorphins ($\alpha$-endorphin, $\beta$-endorphin, $\gamma$-endorphin); dynorphins (dynorphin A, dynorphin B); proenkephalins as the precursors thereof (proenkephalins, propiomelanocortins, prodynorphins and the like), and the like. Among these, opium alkaloids are preferred, and morphine and salts thereof are particularly preferred.

Administration method of the aforementioned medicament of the present invention is not particularly limited, and it can be orally or parenterally administered to human or mammals other than human depending on the type, dosage form and the like of the active ingredient. As the medicament of the present invention, a substance that is a $V_{1b}$ receptor antagonist per se may be used. However, it is usually preferable to add one or more kinds of additives for pharmaceutical preparations as required to the aforementioned substance as the active ingredient to provide the medicament as a preparation in a form available for those skilled in the art. In general, the medicament of the present invention can be administered separately from a narcotic analgesic by using a narcotic analgesic which itself is provided in a dosage form of a solution, tablet or the like in combination. If necessary, however, a pharmaceutical composition (so-called a combined drug) containing a narcotic analgesic and a $V_{1b}$ receptor antagonist as the active ingredient of the medicament of the present invention can also be prepared and administered.

Methods for the combination use with a narcotic analgesic are not particularly limited. Employable methods include, for example, a method of continuously administering the medicament of the present invention throughout the administration period of a narcotic analgesic; a method of administering the medicament of the present invention as required during the administration period of a narcotic analgesic; a method of starting administration of the medicament of the present invention prior to administration of a narcotic analgesic and then continuing administration of the narcotic analgesic and the medicament of the present invention; a method of continuously administering a narcotic analgesic and the medicament of the present invention, then terminating the administration of the narcotic analgesic and further continuing the administration of the medicament of the present invention alone, and the like.

Examples of preparations as dosage units suitable for oral administration include tablets, capsules, powders, subtilized granules, granules, solutions, syrups, and the like. Examples of preparations as an dosage unit suitable for parenteral administration include injections for subcutaneous, intravenous or intramuscular injection, drip infusions, suppositories, inhalants, transdermal agents, transmucosal agents, patches, and the like. Examples of the additives for pharmaceutical preparations include excipients, disintegrating agents or disintegrating aids, binders, lubricants, coating agents, dyes, diluents, vehicles, dissolving agents or dissolving aids, isotonic agents, pH modifiers, stabilizers, propellants, tackifiers, and the like. These additives for pharmaceutical preparations are widely used by those skilled in the art, and it should be recognized that suitable additives for pharmaceutical preparations can be selected for a specific dosage form.

Although dose and administration period of the medicament of the present invention are not particularly limited, they can be selected depending on the type and the administration route of the active ingredient, degree of tolerance development, purpose of administration such as prophylactic or therapeutic use, age and body weight of a patient, and the like. The effectively acting concentration of the $V_{1b}$ receptor antagonist as the active ingredient can be easily confirmed by those skilled in the art by using, for example, the method specifically explained in the following examples. The dose is preferably selected by using the effectively acting concentration as a criterion so that a sufficient blood concentration can be achieved. For example, when a narcotic analgesic such as morphine hydrochloride, morphine nitrate or a sustained-release preparation thereof is administered once to 3 times a day at a dose of about 10 to 30 mg per day, the dose of the medicament of the present invention may be selected to be in the range of about 0.01 to 10,000 mg/day in terms of the amount of the active ingredient. When the medicament of the present invention is repeatedly administered at a large dose, it is desirable to suitably select the dose while monitoring the suppressing action on development of tolerance to analgesic effect. It is preferable to administer the medicament of the present invention as long as possible throughout the administration period of a narcotic analgesic.

Examples

The present invention will be explained more specifically with reference to the following examples. However, the scope of the present invention is not limited to these examples.

Example 1

$V_{1a}$ receptor knockout mice (Neuroscience Letters, 356, pp.195-198, 2004), $V_{1b}$ receptor knockout mice (J. Clin. Invest., 113, pp.302-309, 2004), and control mice (body weight: about 30 g) were subcutaneously (s.c.) given with 10 mg/kg of morphine hydrochloride once a day, which was repeated for 15 days. The analgesic effect was evaluated by the tail-flick test on 1st, 5th, 9th, 12th and 15th days after the administration on the basis of the maximal possible effect (%MPE), which represents analgesic intensity and is calculated in accordance with the following equation.

$$\%\mathrm{MPE} = 100 \times [(\text{Measured value after treatment} - \text{Measured value before treatment})$$
$$+ (\text{Cut-off value} - \text{Measured value before treatment})]$$

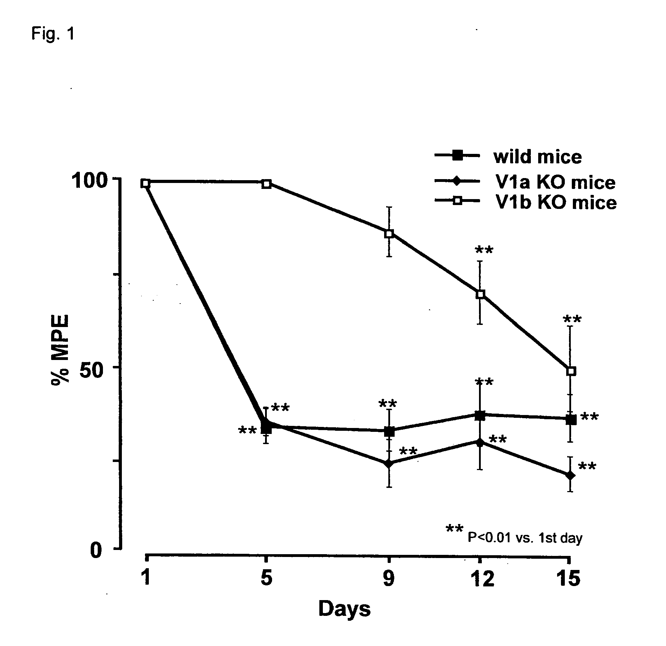

As a result, it was found that tolerance to morphine was developed in the $V_{1a}$ receptor knockout mice and the control mice, whereas remarkable resistance against tolerance to morphine was observed in the $V_{1b}$ receptor knockout mice. The results are shown in Fig. 1. These results suggested that the $V_{1b}$ receptor was involved in development of tolerance to morphine.

Example 2

Male ddY mice (5- or 6-week old) were intracerebroventricularly (i.c.v.) given with 5 $\mu$l of physiological saline or a $V_1$ receptor antagonist (0.5, 5 or 10 ng), and immediately after the administration, the mice were subcutaneously given with 10 mg/kg of morphine hydrochloride, which was repeated twice a day for 5 days to induce tolerance to morphine analgesic. The analgesic effect was evaluated by the tail-flick test on 1st, 3rd and 5th days after the administration on the basis of intensity of analgesic effect using %MPE and AUC (area under the time-reaction curve, Area Under the Curve). As the $V_1$ receptor antagonist, the following three types of antagonists were used.

(a) PhAcALVP ([phenylacetyl, O-Me-D-Try, Arg, Lys]-vasopressin amide): Antagonist highly selective to the $V_{1a}$ receptor
(b) d(CH$_2$)$_5$Tyr(Me)AVP ([β-mercapto-β,β-cyclopentamethylenepropionyl, O-Me-Tyr, Arg]-vasopressin): Antagonist of the $V_{1a}$ receptor
(c) dPenTyr(Me)AVP (deamino-Pen, O-Me-Tyr, Arg]-vasopressin): Non-selective $V_1$ receptor antagonist
Selectivity to the $V_{1a}$ receptor: PhAcALVP > d(CH$_2$)$_5$Tyr(Me)AVP > dPenTyr(Me)AVP
Selectivity to the $V_{1b}$ receptor: dPenTyr(Me)AVP > d(CH$_2$)$_5$Tyr(Me)AVP.

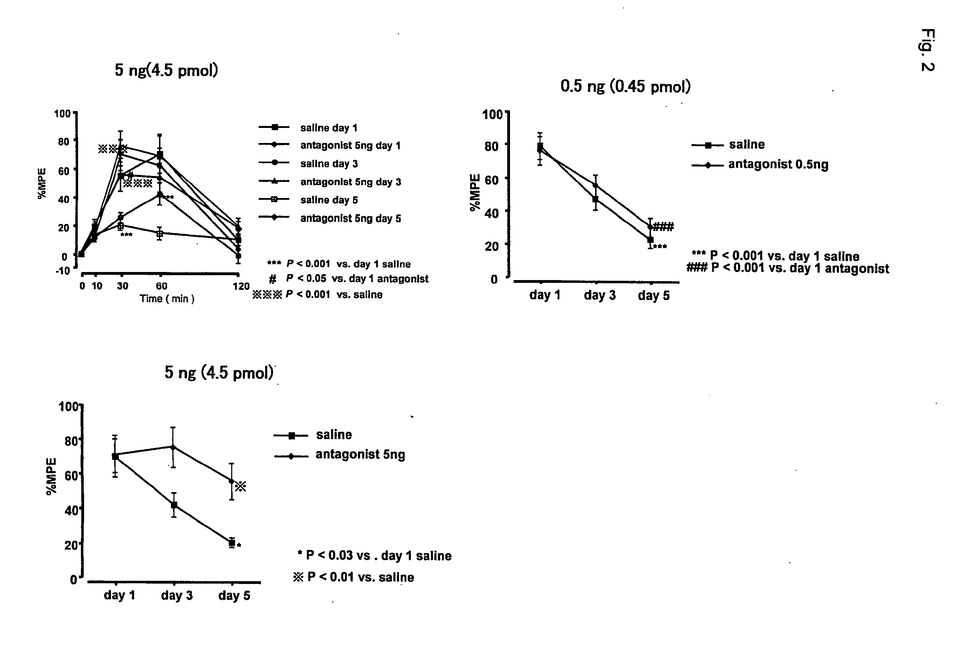

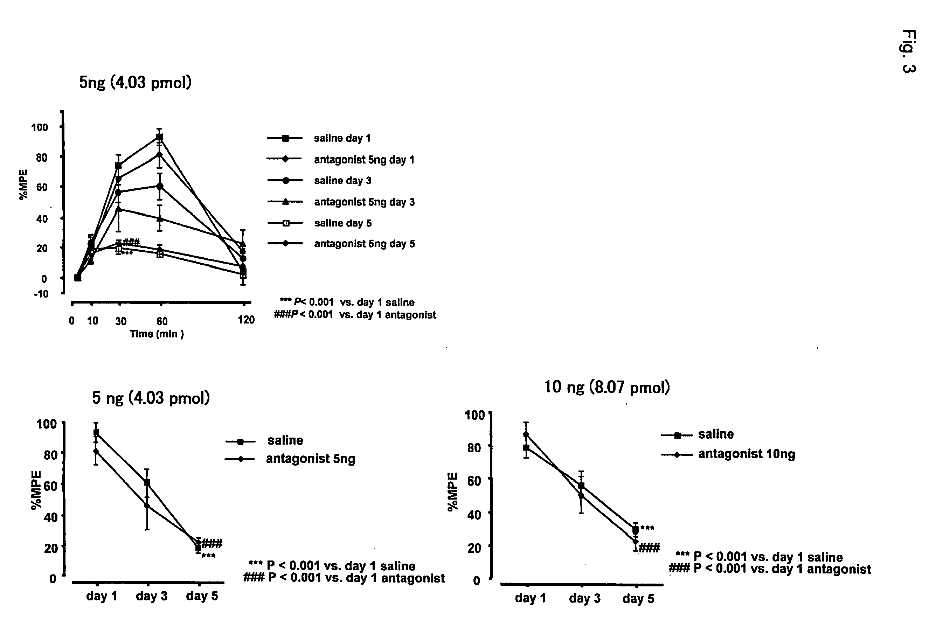

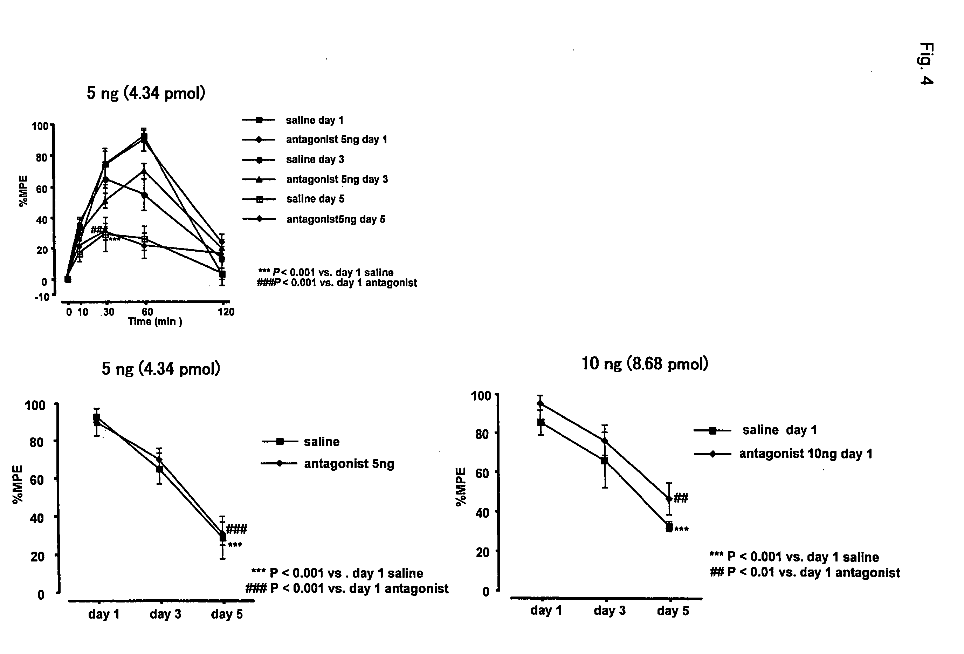

The results are shown in Figs. 2 to 4. PhAcALVP, an antagonist highly selective to the $V_{1a}$ receptor, and d(CH$_2$)$_5$Tyr(Me)AVP, an antagonist of the $V_{1a}$ receptor, had no effect on the tolerance development. Whilst, dPenTyr(Me)AVP, which acts as an antagonist of the $V_{1a}$ receptor and the $V_{1b}$ receptor, suppressed the development of tolerance. These results indicated that agents acting as an antagonist of the $V_{1b}$ receptor suppressed the development of tolerance to morphine.

Example 3

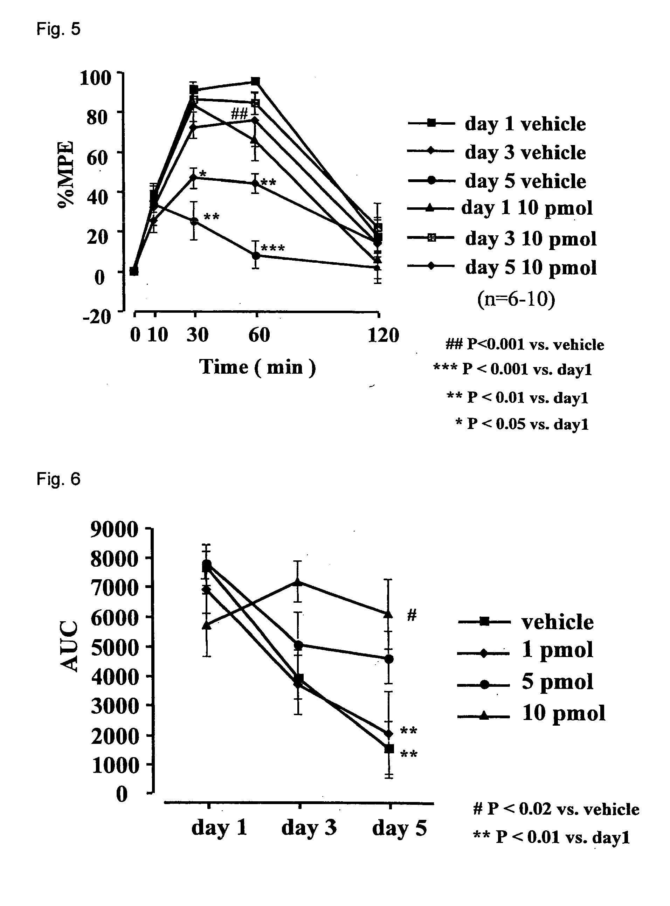

Effect on development of tolerance to morphine-induced analgesic effect was examined by using an antagonist selective to the $V_{1b}$ receptor, (2S,4R)-1-[5-chloro-1-[2,4-dimethoxyphenyl]sulfonyl]-3-(2-methoxyphenyl)-2-oxo-2,3-dihydro-1H-indol-3-yl]-4-hydroxy N,N-dimethyl-2-pyrrolidine carboxamine (SSR149415, The Journal of Pharmacology Experimental Therapeutics, 300, pp.1122-1130, 2002). In combination with morphine (10 mg/kg, s.c.), ddy mice were given with a solvent (1% DMSO in physiological saline, i.c.v) or a $V_{1b}$ antagonist (i.c.v.) twice a day (at 9:00 and 17:00) for 4 days. The analgesic effect was determined by the tail-flick test (TailFlick Unit, UgoBasile, Milano, Italy). The analgesic effect of morphine (10 mg/kg, s.c.) was observed after the first administration of morphine on the 1st, 3rd, and 5th days. Intensity of the heat source was set so that the reference reaction time became 2 or 3 seconds. Cut-off time was set to be 10 seconds so that possible damage to the caudal skin was minimized. The analgesic effect was represented in terms of the maximal possible effect (%MPE) for the time lapsed after the administration of morphine (Fig. 5). The intracerebroventricular administration of the $V_{1b}$ receptor antagonist had no effect on the acute morphine-induced analgesic effect.

Fig. 6 shows effect of a $V_{1b}$ receptor antagonist on the development of tolerance to the morphine-induced analgesic effect. The area under the time-reaction curve (AUC) was obtained using the time lapse shown in Fig. 5. AUC is considered to represent the total analgesic effect level. It was demonstrated that, by intracerebroventricular administration of the $V_{1b}$ receptor antagonist, the development of tolerance to morphine was successfully suppressed without any effect on the acute morphine-induced analgesic effect. Industrial Applicability

The medicament of the present invention has a suppressing action against development of tolerance to analgesic effect induced by administration of a narcotic analgesic such as morphine, and can reduce or prevent development of tolerance to analgesic effect of a narcotic analgesic.

**Claims**

1. A medicament for suppressing development of tolerance to analgesic effect of a narcotic analgesic, which comprises an antagonist of the vasopressin receptor 1b as an active ingredient.

2. The medicament according to claim 1, which is for combination use with a narcotic analgesic.

3. The medicament according to claim 1, wherein the narcotic analgesic is morphine hydrochloride.

Fig. 1

Fig. 2

**Fig. 3**

Fig. 4

Fig. 5

day 1 vehicle
day 3 vehicle
day 5 vehicle
day 1 10 pmol
day 3 10 pmol
day 5 10 pmol
(n=6-10)

## P<0.001 vs. vehicle
*** P < 0.001 vs. day1
** P < 0.01 vs. day1
* P < 0.05 vs. day1

Fig. 6

vehicle
1 pmol
5 pmol
10 pmol

# P < 0.02 vs. vehicle
** P < 0.01 vs. day1

## INTERNATIONAL SEARCH REPORT

| | International application No. |
|---|---|
| | PCT/JP2005/023783 |

A.  CLASSIFICATION OF SUBJECT MATTER
***A61K45/00***(2006.01), ***A61K31/485***(2006.01), ***A61K31/404***(2006.01), ***A61K38/00***
(2006.01), ***A61P25/04***(2006.01)

According to International Patent Classification (IPC) or to both national classification and IPC

B.  FIELDS SEARCHED

Minimum documentation searched (classification system followed by classification symbols)
A61K31/404, A61K31/485, A61K38/00, A61K45/00, A61P25/04

Documentation searched other than minimum documentation to the extent that such documents are included in the fields searched
Jitsuyo Shinan Koho        1922-1996   Jitsuyo Shinan Toroku Koho   1996-2006
Kokai Jitsuyo Shinan Koho  1971-2006   Toroku Jitsuyo Shinan Koho   1994-2006

Electronic data base consulted during the international search (name of data base and, where practicable, search terms used)
CA(STN), BIOSIS(STN), BIOTECHABS(STN), EMBASE(STN), MEDLINE(STN),
SwissProt/PIR/Geneseq, PubMed

C.  DOCUMENTS CONSIDERED TO BE RELEVANT

| Category* | Citation of document, with indication, where appropriate, of the relevant passages | Relevant to claim No. |
|---|---|---|
| X | XU, Q. et al., Dependency on the brain function of arginine vasopressin system of the development to and recovery from analogesic tolerance to morphine. Brain.Res., 1991, Vol.577, pages 189 to 193, 189 to 191, Fig. 3 | 1-4 |
| X | TAO, L.T. et al., The roel of vasopressin on the effect of U-50,448 to block the development of morphine tolerance and physical dependence. Naunyn-Schmiedeberg's Arch.Pharmacol., 1997, Vol.335, pages 281 to 287, 281 to 283, Fig. 4 | 1-4 |
| X | SU, M.T. et al., Blockade of the development of morphine tolerance by U-50,448, an AVP antagonist or MK801 in the rat hipopocampal slice.Br.J.Pharmacol., 1998, Vol.123, pages 625 to 630, 625 to 627, Fig. 4 | 1-4 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered    to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 23 January, 2006 (23.01.06) | 31 January, 2006 (31.01.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

| International application No. |
| --- |
| PCT/JP2005/023783 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | GAL, C.S. et al., Characterization of (2S,4R)-1-[5-Chloro-1-[(2,4-dimethoxyphenyl)sulfonyl]-3-(2-methoxyphenyl)-2-oxo-2,3-dihydro-1H-indol-3-yl]-4-hydroxy-N,N-dimethyl-2-pyrrolidine carboxamide(SSR149415), a selective and orally active vasopressin $V_{1b}$ receptor antagonist. J.Pharmacol.Exp.Ther., 2002, Vol.300, No.3, pages 1122 to 1130, 1123, 1125, Fig. 2 | 1-4 |
| A | SUGIMOTO, T. et al., Molecular cloning and functional expression of a cDNA encoding the human $V_{1b}$ vasopressin receptor. J.Biol.Chem., 1994, Vol.269, No.43, pages 27088 to 27092, 27091, Fig.2 | 1-4 |
| A | ANTONI, F.A. et al., Novel ligand specificity of pituitary vasopressin receptors in the rat. Neuroendocrinology, 1994, Vol.39, pages 186 to 188, abstract, Table 1 | 1-4 |
| A | RIVER, C. et al., Studies of the nature of the interaction between vasopressin and corticotrophin-releasing factor on adrenocorticotropin release in the rat. Endocrinology, 1984, Vol.115, No.3, pages 882 to 886, 883, Fig. 1, 2 | 1-4 |
| A | RENE, P. et al., Nucleotide sequence and structural prganization of the human vasopressin pituitary receptor (V3) gene. Gene, 2000, Vol.241, pages 57 to 64, abstract | 1-4 |
| A | VENTURA, M.A. et al., Gene and cDNA cloning and characterization of the mouse V3/V1b pituitary vasopressin receptor. J.Mol. Endocrinol., 1999, Vol.22, pages 251 to 260, abstract | 1-4 |

Form PCT/ISA/210 (continuation of second sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 976139 A
- JP 2003523351 A
- JP 2003523354 A
- JP 2003525287 A
- JP 2004050265 A
- JP 2004502654 A

**Non-patent literature cited in the description**

- **Y. DE KEYSER et al.** *Febs Letters,* 1994, vol. 356, 215-220
- **C.S-L., GAL.** *J. Pharm. Exp. Ther.,* 2002, vol. 300, 1122-1130
- *Neuroscience Letters,* 2004, vol. 356, 195-198
- *J. Clin. Invest.,* 2004, vol. 113, 302-309
- *The Journal of Pharmacology Experimental Therapeutics,* 2002, vol. 300, 1122-1130